# EUROPEAN PATENT APPLICATION

(11) **EP 4 678 658 A1**
(43) Date of publication of application: **14.01.2026**
(21) Application number: 24767088.8
(22) Date of filing: 01.03.2024
(51) Int. Cl.: C07K 16/18, C07K 17/00, C12N 1/15, C12N 1/19, C12N 1/21, C12N 5/10, C12N 15/13, C12N 15/63, C12P 21/08

(54) **ANTIBODY OR ANTIGEN-BINDING FRAGMENT OF SAME**

(30) Priority: 03.03.2023 JP 2023033015
(71) Applicant: KARYDO THERAPEUTIX, INC., Tokyo 102-0082 (JP)
(72) Inventor: SATO, Narutoku, Soraku-gun, Kyoto 619-0288 (JP)
(74) Representative: Marks & Clerk LLP
(86) International application number: PCT/JP2024/007913
(87) International publication number: WO 2024/185720

(57) **Abstract**

An object of the present invention is to provide an antibody capable of detecting a human PRB 1 protein not only in saliva but also in serum.

The problem is solved by the antibody or the antigen-binding fragment thereof having a capability of binding to the human PRB1 protein, wherein a heavy chain variable region includes an amino acid sequence that is 95% or more identical to a heavy chain variable region represented by an amino acid sequence from 19th to 132nd positions in SEQ ID NO: 1, and a light chain variable region includes an amino acid sequence that is 95% or more identical to a light chain variable region represented by an amino acid sequence from 21st to 132nd positions in SEQ ID NO: 6.

## Description

### Technical Field

The present description discloses an antibody or an antigen-binding fragment thereof having a capability of binding to a human PRB1 protein.

### Background Art

Patent Literatures 1 describes a use of measurements of PRB1 protein in salivary glands and saliva as a marker of renal function.

Patent Literatures 2 describes that in a mouse model of chronic renal failure, measurements of the PRB1 protein in the salivary glands and the saliva increase as the renal function declines.

### Citation List

### Patent Literature

Patent Literature 1: US 2019/0033325 A1
Patent Literature 2: US 2019/0218587 A1

### Summary of Invention

### Technical Problem

An object of the present invention is to provide an antibody capable of detecting human PRB1 protein not only in saliva but also in serum.

### Solution to Problem

The present disclosure may include the following embodiments.

### Item 1.

An antibody or an antigen-binding fragment thereof having a capability of binding to a human PRB1 protein, wherein
a heavy chain variable region includes an amino acid sequence that is 95% or more identical to a heavy chain variable region represented by an amino acid sequence from 19th to 132nd positions in SEQ ID NO: 1, and
a light chain variable region includes an amino acid sequence that is 95% or more identical to a light chain variable region represented by an amino acid sequence from 21st to 132nd positions in SEQ ID NO: 6.

Item 2. The antibody or the antigen-binding fragment thereof according to item 1, wherein
the heavy chain variable region includes
a heavy chain CDR1 consisting of an amino acid sequence represented by SEQ ID NO: 3,
a heavy chain CDR2 consisting of an amino acid sequence represented by SEQ ID NO: 4, and
a heavy chain CDR3 consisting of an amino acid sequence represented by SEQ ID NO: 5, and
the light chain variable region includes,
a light chain CDR1 consisting of an amino acid sequence represented by SEQ ID NO: 8,
a light chain CDR2 consisting of an amino acid sequence represented by SEQ ID NO: 9, and
a light chain CDR3 consisting of an amino acid sequence represented by SEQ ID NO: 10.

### Item 3.

A polynucleotide encoding a polypeptide including a heavy chain variable region including an amino acid sequence that is 95% or more identical to a heavy chain variable region represented by an amino acid sequence from 19th to 132nd positions in SEQ ID NO: 1.

### Item 4.

The polynucleotide according to item 3, wherein
the heavy chain variable region includes
a heavy chain CDR1 consisting of an amino acid sequence represented by SEQ ID NO: 3,
a heavy chain CDR2 consisting of an amino acid sequence represented by SEQ ID NO: 4, and
a heavy chain CDR3 consisting of an amino acid sequence represented by SEQ ID NO: 5.

### Item 5.

A vector including the polynucleotide according to item 3.

### Item 6.

A vector including the polynucleotide according to item 4.

### Item 7.

A cell expressing a polypeptide including a heavy chain variable region including an amino acid sequence that is 95% or more identical to a heavy chain variable region represented by an amino acid sequence from 19th to 132nd positions in SEQ ID NO: 1.

### Item 8.

The cell according to item 7, wherein
the heavy chain variable region includes
a heavy chain CDR1 consisting of an amino acid sequence represented by SEQ ID NO: 3,
a heavy chain CDR2 consisting of an amino acid sequence represented by SEQ ID NO: 4, and
a heavy chain CDR3 consisting of an amino acid sequence represented by SEQ ID NO: 5.

### Item 9.

A polynucleotide encoding a polypeptide including a light chain variable region including an amino acid sequence that is 95% or more identical to a light chain variable region represented by an amino acid sequence from 21st to 132nd positions in SEQ ID NO: 6.

### Item 10.

The polynucleotide according to item 9, wherein
the light chain variable region includes
a light chain CDR1 consisting of an amino acid sequence represented by SEQ ID NO: 8,
a light chain CDR2 consisting of an amino acid sequence represented by SEQ ID NO: 9, and
a light chain CDR3 consisting of an amino acid sequence represented by SEQ ID NO: 10.

### Item 11.

A vector including the polynucleotide according to item 9.

### Item 12.

A vector including the polynucleotide according to item 10.

### Item 13.

A cell expressing a polypeptide including a light chain variable region including an amino acid sequence that is 95% or more identical to a light chain variable region represented by an amino acid sequence from 21st to 132nd positions in SEQ ID NO: 6.

### Item 14.

The cell according to item 13, wherein
the light chain variable region includes
a light chain CDR1 consisting of an amino acid sequence represented by SEQ ID NO: 8,
a light chain CDR2 consisting of an amino acid sequence represented by SEQ ID NO: 9, and
a light chain CDR3 consisting of an amino acid sequence represented by SEQ ID NO: 10.

### Item 15.

The antibody or the antigen-binding fragment thereof according to item 1 or 2 labeled with a labeling substance.

### Item 16.

A solid phase on which an antibody or an antigen-binding fragment thereof according to item 1 or 2 is immobilized.

### Advantageous Effects of Invention

The human PRB1 protein in the serum can be detected.

### Brief Description of Drawings

FIG. 1 shows sequences of each region of a monoclonal antibody 8A10 clone.
FIG. 2 shows sequences of each region of a monoclonal antibody 2B3 clone.
FIG. 3 shows a calibration curve for measuring the concentration of a human PRB1 protein in saliva.
FIG. 4 shows concentrations of the human PRB1 protein in saliva from each subject.
FIG. 5 shows a calibration curve for measuring the concentration of the human PRB1 protein in serum.
FIG. 6 shows concentrations of the human PRB1 protein in serum from each subject.
FIG. 7 shows results of detection of the hPRB1 in human saliva and serum samples.
FIG. 8 shows results of analysis of an association between the hPRB1 and human CKD. FIG. 8a shows results of analysis using a LightGBM classifier. FIG. 8b shows results of analysis using a logistic regression model.

### Description of Embodiments

### 1. Antibody or antigen-binding fragment thereof

One embodiment of the present invention relates to an antibody or an antigen-binding fragment thereof binding to a human PRB 1 protein (hereinafter also referred to as "hPRB1").

In the present description, the antibodies of any class are intended to be a single Y-shaped unit in which a heavy chain and a light chain are associated. The antibodies include classes of IgG, IgM, IgA, IgD, and IgE. In addition, the Y-shaped unit of the antibody may include subclasses depending on the heavy chain. If the class of the antibody is the IgG, the subclass includes one selected from four subclasses of IgG₁, IgG₂ₐ, IgG_{2b}, IgG₃, and IgG₄. If the class of the antibody is the IgA, the subclass includes at least one selected from two subclasses of IgA1 and IgA2.

The antibody may be in a multimeric structure with 2 to 5 Y-shaped units bound together. In particular, if the antibody is the IgM, it may be in a pentameric structure.

The light chain of the antibody may be a lambda chain or a kappa chain.

The antibody may be obtained from, for example, a rat, mouse, rabbit, goat, guinea pig, pig, chicken, ostrich, or the like.

In the present invention, a first antibody has a capability of binding to a human PRB1 protein. A heavy chain variable region of the first antibody includes an amino acid sequence that is 95% or more identical to a heavy chain variable region represented by an amino acid sequence from 19th to 132nd positions in SEQ ID NO: 1. In addition, a light chain variable region of the first antibody includes an amino acid sequence that is 95% or more identical to a light chain variable region represented by an amino acid sequence from 21st to 132nd positions in SEQ ID NO: 6.

The identity of each variable region is preferably 96% or more, 97% or more, 98% or more, 99% or more, or 100%. An amino acid sequence of a non-identical part may include substitutions, deletions, or insertions of amino acid residues for the amino acid sequence of the heavy chain variable region represented by the amino acid sequence from 19th to 132nd positions in SEQ ID NO: 1 and/or the light chain variable region represented by the amino acid sequence from 21st to 132nd positions in SEQ ID NO: 6.

For the first antibody, more preferably, the heavy chain variable region includes
a heavy chain CDR1 consisting of an amino acid sequence represented by SEQ ID NO: 3,
a heavy chain CDR2 consisting of an amino acid sequence represented by SEQ ID NO: 4, and
a heavy chain CDR3 consisting of an amino acid sequence represented by SEQ ID NO: 5,
and the light chain variable region includes
a light chain CDR1 consisting of an amino acid sequence represented by SEQ ID NO: 8,
a light chain CDR2 consisting of an amino acid sequence represented by SEQ ID NO: 9, and
a light chain CDR3 consisting of an amino acid sequence represented by SEQ ID NO: 10.

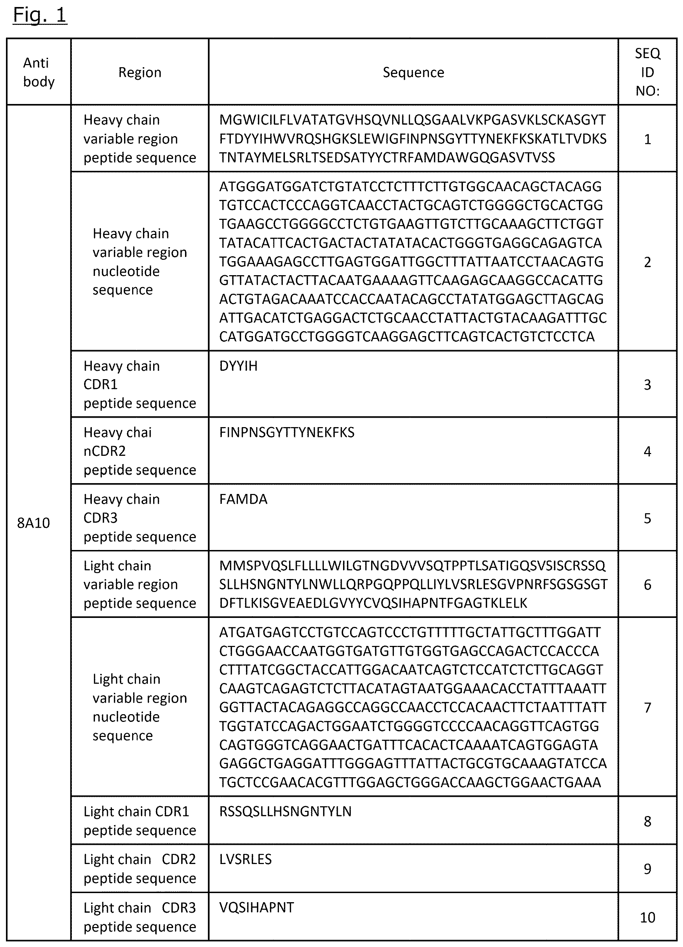

FIG. 1 shows a more preferred embodiment of the first antibody. The subclass of the first antibody is preferably IgG₁, and the light chain is the kappa chain.

In the present invention, a second antibody has the capability of binding to the human PRB 1 protein. A heavy chain variable region of the second antibody includes an amino acid sequence that is 95% or more identical to a heavy chain variable region represented by an amino acid sequence from 21st to 141st positions in SEQ ID NO: 11. A light chain variable region of the second antibody includes an amino acid sequence that is 95% or more identical to a light chain variable region represented by an amino acid sequence from 26th to 132nd positions in SEQ ID NO: 16.

The identity of each variable region is preferably 96% or more, 97% or more, 98% or more, 99% or more, or 100%. An amino acid sequence of a non-identical part may include substitutions, deletions, or insertions of amino acid residues for the amino acid sequence of the heavy chain variable region represented by the amino acid sequence from 21st to 141st positions in SEQ ID NO: 11 and/or the light chain variable region represented by the amino acid sequence from 26th to 132nd positions in SEQ ID NO: 16.

For the second antibody, more preferably, the heavy chain variable region includes
a heavy chain CDR1 consisting of an amino acid sequence represented by SEQ ID NO: 13,
a heavy chain CDR2 consisting of an amino acid sequence represented by SEQ ID NO: 14, and
a heavy chain CDR3 consisting of an amino acid sequence represented by SEQ ID NO: 15,
and the light chain variable region includes
a light chain CDR1 consisting of an amino acid sequence represented by SEQ ID NO: 18,
a light chain CDR2 consisting of an amino acid sequence represented by SEQ ID NO: 19, and
a light chain CDR3 consisting of an amino acid sequence represented by SEQ ID NO: 20.

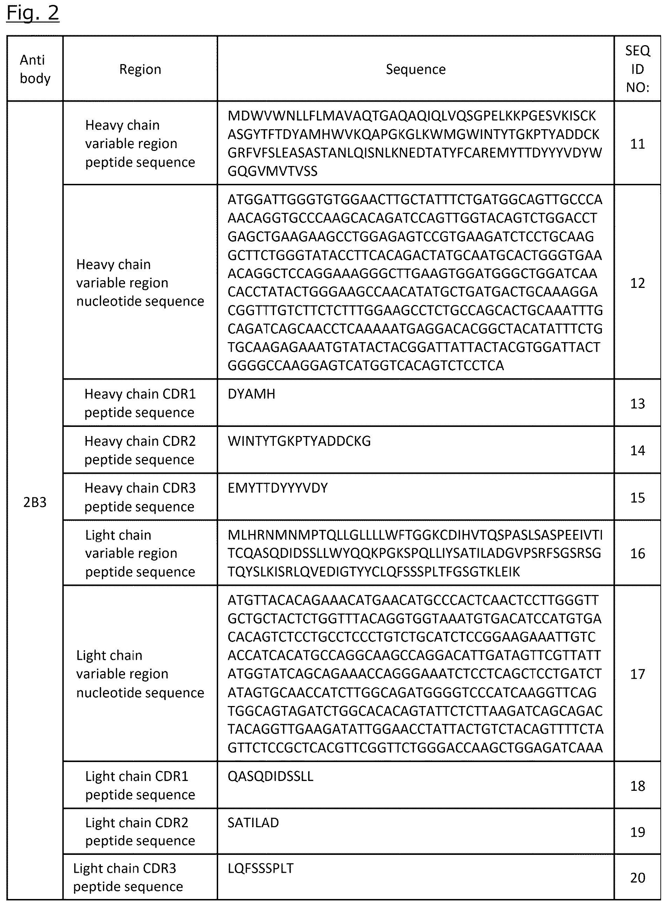

FIG. 2 shows a more preferred embodiment of the second antibody. The class of the second antibody is preferably IgM, and the light chain is the kappa chain.

The antigen-binding fragment of the antibody is not limited as long as it includes the heavy chain variable region and the light chain variable region described above. The antigen-binding fragment may include F(ab'), Fab, F(ab')₂, single chain Fv fragment (scFv), and the like.

The antibody or the antigen-binding fragment thereof may be labeled with a labeling substance.

The labeling substance may include, for example, avidin, streptavidin, biotin, a substance that generates a detectable signal, and the like. For example, the substance that generates the detectable signal may be a substance that itself generates the signal (hereinafter referred to as "signal-generating substance") or a substance that catalyzes a reaction of other substances to generate the signal. The signal-generating substance may include, for example, a fluorescent substance, radioisotope, and the like.

The fluorescent substance may include, for example, a fluorescent dye such as fluorescein isothiocyanate (FITC), Rhodamine, and Alexa Fluor^{®}, a fluorescent protein such as Green Fluorescent Protein (GFP), Blue Fluorescent Protein (BFP), Cyan Fluorescent Protein (CFP), Yellow Fluorescent Protein (YFP), and Red Fluorescent Protein (RFP), and the like. The radioisotope may include, for example, ¹²⁵I, ¹⁴C, ³²P, and the like.

The substance that catalyzes the reaction of other substances to generate the detectable signal may include, for example, an enzyme. The enzymes may include alkaline phosphatase, peroxidase, beta-galactosidase, luciferase, and the like.

The antibody or the antigen-binding fragment thereof may be immobilized on a solid phase. The solid phase may include, for example, microplates, beads, and the like. Materials for the solid phase are not particularly limited, and may be selected from for example, organic polymeric compounds, inorganic compounds, biopolymers, and the like. The organic polymeric compounds may include latex, polystyrene, polypropylene, and the like. The inorganic compounds may include magnetic materials (such as iron oxide, chromium oxide, and ferrite), silica, alumina, glass, and the like. The biopolymers may include insoluble agarose, insoluble dextran, gelatin, cellulose, and the like.

The antibody or the antigen-binding fragment thereof may be attached with a tag. The tags may include a histidine tag, a FLAG^{®} tag, a hemagglutinin (HA) tag, a glutathione S-transferase (GST) tag, a myc tag, and the like.

### 2. Polypeptide, polynucleotide, and vector including polynucleotide

One embodiment of the present invention relates to a polypeptide including part or all of the antibody or the binding fragment thereof, and a polynucleotide encoding the polypeptide.

The polypeptide includes the amino acid sequence that is 95% or more identical to the heavy chain variable region and/or the light chain variable region described in the above section 1, and the polynucleotide encodes the polypeptide.

When the antibody is the first antibody, the polynucleotide encodes the polypeptide including the heavy chain variable region including the amino acid sequence that is 95% or more identical to the heavy chain variable region represented by the amino acid sequence from 19th to 132nd positions in SEQ ID NO: 1.

The polynucleotide encodes the polypeptide including the light chain variable region including the amino acid sequence that is 95% or more identical to the light chain variable region represented by the amino acid sequence from 21st to 132nd positions in SEQ ID NO: 6.

Preferably, when the antibody is the first antibody, the polynucleotide including the heavy chain variable region encodes the polypeptide including
the heavy chain CDR1 consisting of the amino acid sequence represented by SEQ ID NO: 3,
the heavy chain CDR2 consisting of the amino acid sequence represented by SEQ ID NO: 4, and
the heavy chain CDR3 consisting of the amino acid sequence represented by SEQ ID NO: 5.

In addition, when the antibody is the first antibody, the polynucleotide including the light chain variable region encodes the polypeptide including
the light chain CDR1 consisting of the amino acid sequence represented by SEQ ID NO: 8,
the light chain CDR2 consisting of the amino acid sequence represented by SEQ ID NO: 9, and
the light chain CDR3 consisting of the amino acid sequence represented by SEQ ID NO: 10.

When the antibody is the second antibody, the polynucleotide encodes the polypeptide including the heavy chain variable region including the amino acid sequence that is 95% or more identical to the heavy chain variable region represented by the amino acid sequence from 21st to 141st positions in SEQ ID NO: 11.

The polynucleotide encodes the polypeptide including the light chain variable region including the amino acid sequence that is 95% or more identical to the light chain variable region represented by the amino acid sequence from 26th to 132nd positions in SEQ ID NO: 16.

Preferably, when the antibody is the second antibody, the polynucleotide including the heavy chain variable region encodes the polypeptide including
the heavy chain CDR1 consisting of the amino acid sequence represented by SEQ ID NO: 13,
the heavy chain CDR2 consisting of the amino acid sequence represented by SEQ ID NO: 14, and
the heavy chain CDR3 consisting of the amino acid sequence represented by SEQ ID NO: 15.

In addition, when the antibody is the second antibody, the polynucleotide including the light chain variable region encodes the polypeptide including
the light chain CDR1 consisting of the amino acid sequence represented by SEQ ID NO: 18,
the light chain CDR2 consisting of the amino acid sequence represented by SEQ ID NO: 19, and
the light chain CDR3 consisting of the amino acid sequence represented by SEQ ID NO: 20.

The polypeptide including the heavy chain variable region and the polypeptide including the light chain variable region may each be encoded on different polynucleotide chains or on one polynucleotide chain.

The polynucleotide may be DNA or RNA, preferably DNA. In addition, the polynucleotide may be single-stranded or double-stranded.

The said polynucleotide may be inserted into the vector. The vector may be a cloning vector or an expression vector. The vector may also be a plasmid vector, viral vector, transposon vector, and the like.

### 3. Cell expressing polypeptide

One embodiment of the present invention relates to a cell expressing the polypeptide described in the above section 2. The cell may include a hybridoma. The cell may also include other cells such as E. coli, yeast, mammalian cells (for example, cultured cells such as HEK293, CHO, and the like), avian cells such as chicken cells, insect cells, and the like.

The cell may separately express the polypeptide including the heavy chain variable region and the polypeptide including the light chain variable region described in the above section 2. The cell may also express both the polypeptide including the heavy chain variable region and the polypeptide including the light chain variable region.

### 4. Application of antibody or antigen-binding fragment thereof

The antibody or the antigen-binding fragment described in the above section 1 may be used to detect the human PRB1 protein using an immunological method. The immunological method may include, for example, enzyme-linked immunosorbent assay (ELISA), Western blotting, and immunostaining.

### Examples

The present invention will be described in more detail below with reference to examples. However, the present invention should not be construed as being limited to the examples.

### 1. Preparation of monoclonal antibody

A monoclonal antibody was prepared according to a known method. Specifically, the human PRB1 (NCBI Reference Sequence ID: NP_005030.2) was forcibly expressed in Exp293F cells by transfection, and a recombinant protein (hereinafter also referred to as "hPRB1 recombinant protein" or "recombinant protein") was purified from a culture supernatant. Purification of the recombinant protein was outsourced to Protein Express, Co. Ltd.

According to a known protocol, a rat was inoculated with the hPRB1 recombinant protein, and after a certain period of time, mononuclear cells in ascites fluid were isolated and hybridomas were generated to obtain 8A10 clones (IgG₁) and 2B3 clones (IgM) producing anti-hPRB1 antibody. Preparation of the antibody was outsourced to BioGate, Co. Ltd.

Purification of the antibody was performed using an immunocolumn according to a known method, and the resulting antibody was dissolved in PBS with 0.05% NaN₃ to obtain an antibody stock solution. The monoclonal antibody obtained from the 8A10 clone is hereinafter referred to as "Rat anti-hPRB1 IgG(8A10) mAb" or "8A10 mAb". In addition, the monoclonal antibody obtained from the 2B3 clone is referred to as "Rat anti-hPRB1 IgM(2B3) mAb" or "2B3 mAb". The amino acid sequence of the variable region of each antibody was determined according to Kabat scheme.

### 2. ELISA measurement

### 2-1. Preparation of standard solution and sample

To prepare a calibration curve, KAICO-hPRB1 recombinant protein was diluted with blocking buffer to 0 ng/mL, 0.03125 ng/mL, 0.0625 ng/mL, 0.125 ng/mL, 0.25 ng/mL, 0.5 ng/mL, 1 ng/mL, 2 ng/mL, 3 ng/mL, 4 ng/mL, 6 ng/mL, 8 ng/mL, and 10 ng/mL, and used as a standard solution. The standard solution of 0 ng/mL was used as a blank.

After rinsing the mouth with water about 30 minutes prior to collection, a cotton swab was placed in the mouth of the subject, the mouth was lightly closed, and the tongue was moved from side to side for 30 seconds to absorb the saliva into the cotton part of the cotton swab. The cotton part with the absorbed saliva was immersed in a preservation solution (0.15M Sodium Citrate buffer pH 4.0) for about 30 seconds, and then the cotton swab was removed. The preservation solution in which the cotton part had been immersed was used as a saliva specimen.

Serum was collected from the subject according to a usual method.

The saliva specimen was diluted 100 times in the blocking buffer and used as a measurement sample. A serum specimen was diluted 50 times in the blocking buffer and used as a measurement sample.

### 2-2. ELISA measurement

The Rat anti-hPRB1 IgM (2B3) mAb was diluted in Sodium Carbonate-bicarbonate buffer (0.01M, pH 9.6±0.2) to a final concentration of 10 µg/ml. 50 µL of the diluted 2B3 mAb was added to each well of ELISA plate (IWAKI, 3801-096). After the addition, the plate was sealed with plate seal (stem, P96T01N) and further shaded with an aluminum foil, and incubated by standing overnight at 4°C.

Subsequently, the diluted antibody solution in the ELISA plate after the incubation was aspirated off using an aspirator. Then, 50 µL of blocking buffer (PBS-T (PBS with Tween 20 added so that the Tween 20 is 0.05%) containing 0.2% bovine serum albumin (BSA)) was added to each well. The BSA used was IgG-Free and Protease-Free available from Jakson ImmunoResearch Laboratories Inc. under product number 001-000-161. The plate after the addition was sealed with the plate seal and further shaded with the aluminum foil and then incubated by standing for 2 hours at room temperature.

Subsequently, each well was washed 5 times with the PBS in the ELISA plate after the incubation.

Next, the standard solution for the calibration curve and the measurement sample were added in 50 µL each into separate wells. The plate after the addition was sealed with the plate seal and further shaded with the aluminum foil and then incubated by standing for 1 hour at room temperature.

Subsequently, each well was washed 5 times with the PBS in the ELISA plate after the incubation.

Next, 50 µL of the Rat anti-hPRB1 IgG(8A10) mAb, diluted in the blocking buffer to a final concentration of 10 µg/ml, was added to the wells after the washing. The plate after the addition was sealed with the plate seal and further shaded with the aluminum foil and then incubated by standing for 1 hour at room temperature.

Subsequently, each well was washed 5 times with the PBS in the ELISA plate after the incubation.

Next, 50 µL of F(ab')2 Goat anti-Rat IgG-Fc Fragment Antibody Biotinylated (BETHYL, A110-139B), diluted in the blocking buffer to a final concentration of 0.18 µg/ml, was added to the wells after the washing. The plate after the addition was sealed with the plate seal and further shaded with the aluminum foil and then incubated by standing for 1 hour at room temperature.

Subsequently, each well was washed 5 times with the PBS in the ELISA plate after the incubation.

Next, 50 µL of Avidin protein HRP (Thermo Fisher Scientific, 21123, 5.5 mg/ml) diluted 20,000 times in the blocking buffer was added to the wells after the washing. The plate after the addition was sealed with the plate seal and further shaded with the aluminum foil and then incubated by standing for 1 hour at room temperature.

Subsequently, each well was washed 5 times with the PBS in the ELISA plate after the incubation.

Next, 50 µL of TMB Chromogen Solution (for ELISA) (Thermo Fisher Scientific, 002023) was added to the wells after the washing, and shaded with the aluminum foil and then incubated by standing for 5 minutes at room temperature. 50 µL of 2M H₂SO₄ was added to the wells to stop a chromogenic reaction, and absorbance at 450 nm was then measured in a plate reader (Thermo Fisher Scientific, Multiskan GO) within 10 minutes.

A curve of the calibration curve and a regression equation were created by subtracting an absorbance value of the blank from an absorbance value of a calibration curve sample. For each measurement sample, a value obtained by subtracting the absorbance value of the blank from an absorbance value of the measurement sample was substituted into the calibration curve, and the resulting value was multiplied by the dilution ratio of the measurement sample to calculate the concentration of the hPRB1 in the specimen.

### 3. Results

Measurements of the hPRB1 in the saliva specimen and the hPRB1 in the serum specimen were performed independently, and the calibration curves were prepared each time.

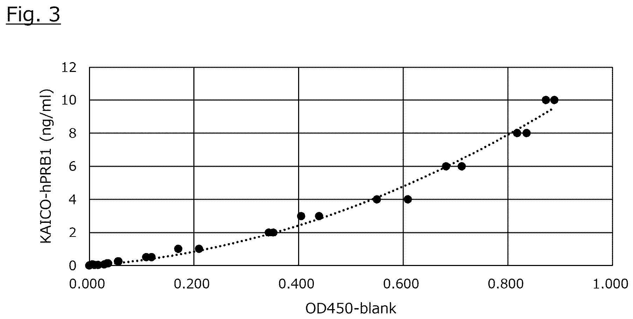

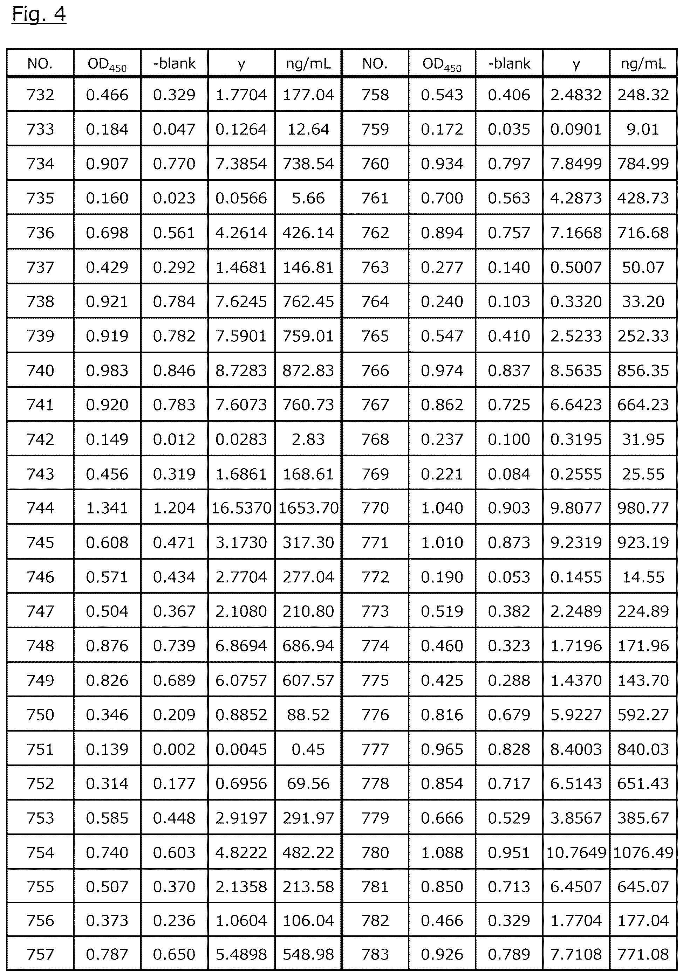

FIG. 3 shows the calibration curve for the measurement of the hPRB1 in the saliva. The regression equation was y = 9.5475x² + 2.2399x and R² = 0.9902. FIG. 4 shows the concentration of the hPRB1 in the saliva collected from each subject. In the figure, "NO." indicates the identification number of each subject, "OD₄₅₀" indicates an actual absorbance value measured in the ELISA measurement, and "-blank" indicates the value obtained by subtracting the absorbance value of the blank from the actual absorbance value measured in the ELISA measurement, "y" indicates a value of y (the concentration of the hPRB1 in the measurement sample) calculated by the regression equation, and "ng/mL" indicates the concentration of the hPRB1 in the specimen (saliva) obtained by multiplying the value of y by the dilution ratio of the specimen.

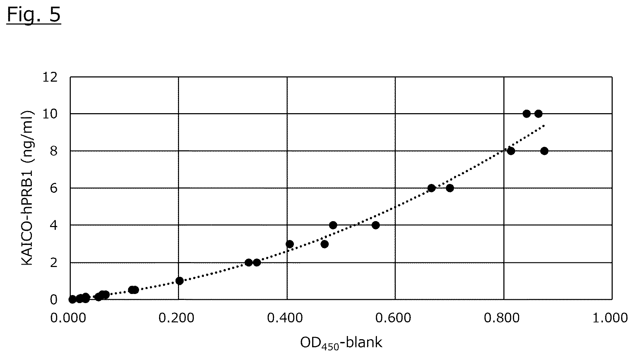

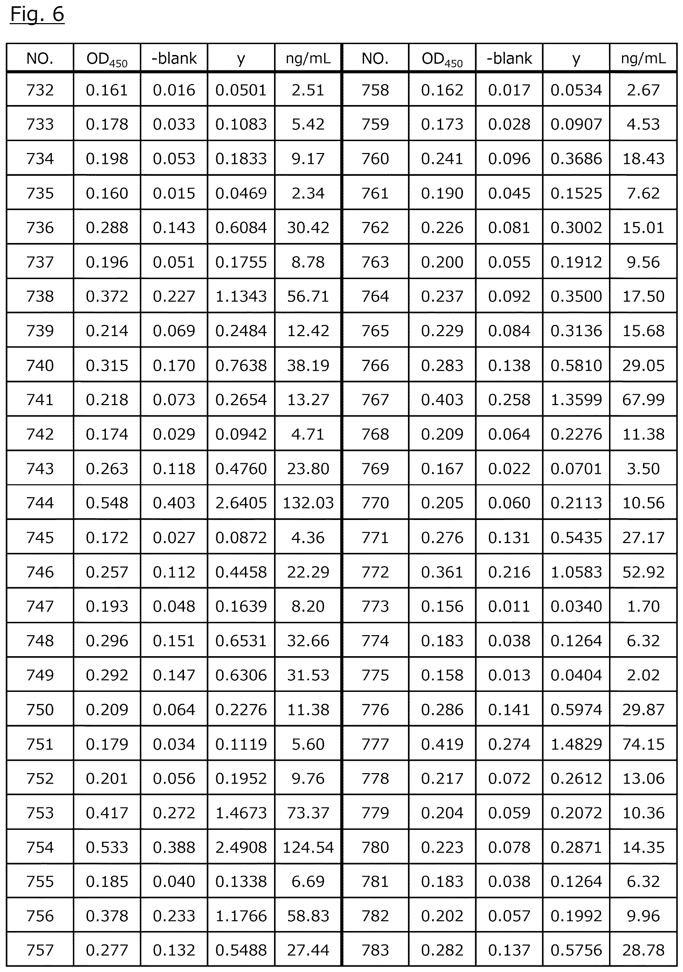

FIG. 5 shows the calibration curve for the measurement of the hPRB1 in the serum. The regression equation was y = 8.8369x² + 2.9909x and R² = 0.9815. FIG. 6 shows the concentration of the hPRB1 in the serum collected from each subject. In the figure, "NO." indicates the identification number of each subject, "OD₄₅₀" indicates an actual absorbance value measured in the ELISA measurement, and "-blank" indicates the value obtained by subtracting the absorbance value of the blank from the actual absorbance value measured in the ELISA measurement, "y" indicates a value of y (the concentration of the hPRB1 in the measurement sample) calculated by the regression equation, and "ng/mL" indicates the concentration of the hPRB1 in the specimen (serum) obtained by multiplying the value of y by the dilution ratio of the specimen.

It was shown that an ELISA system using the 8A10 mAb and the 2B3 mAb can detect the hPRB1 in the saliva and in the serum.

### 4. Detection of hPRB1 in human saliva and serum samples

The hPRB1 in the human saliva sample and the human serum sample was detected by the ELISA.

The Rat anti-hPRB1 IgM(2B3) mAb was diluted in the Sodium Carbonate-bicarbonate buffer (0.01M, pH 9.6±0.2) to a final concentration of 10 µg/ml and used as a capture antibody solution. The capture antibody solution was added to each well of the 96-well plate, the wells were coated overnight at 4°C, and the coated wells were blocked with blocking buffer for 2 hours. The blocking buffer was prepared by dissolving bovine serum albumin (BSA, Jackson ImmunoResearch) to 0.2% in the PBS-T (PBS containing 0.05% Tween-20). After the blocking was completed, the blocking buffer was removed and the standard solution of the hPRB 1 and the samples (diluted saliva, serum, or plasma) were added to the wells and incubated for 1 hour. After the incubation was completed, each well was washed with the PBS-T, followed by addition of the Rat anti-hPRB1 IgG (8A10) mAb diluted to 10 µg/mL in the blocking buffer and incubated for 1 hour. After the incubation was completed, each well was washed again with the PBS-T, and the F(ab')2 Goat anti-Rat IgG-Fc Fragment Antibody, Biotinylated (BETHYL) was added to each well and incubated for 1 hour. After washing each well with the PBS-T, the Avidin protein HRP (Thermo Fisher Scientific) was added to each well and incubated for 1 hour. After washing each well, the TMB Chromogen Solution (Thermo Fisher Scientific) was added. Color change due to the TMB was observed within 5 minutes, and the reaction was stopped by adding 2M H₂SO₄ to each well. Finally, the absorbance of each well was measured at 450 nm using Multiskan GO microplate reader (Thermo Fisher Scientific).

Total protein concentration was quantified using TaKaRa Bradford Protein Assay Kit (Takara Bio, Inc.).

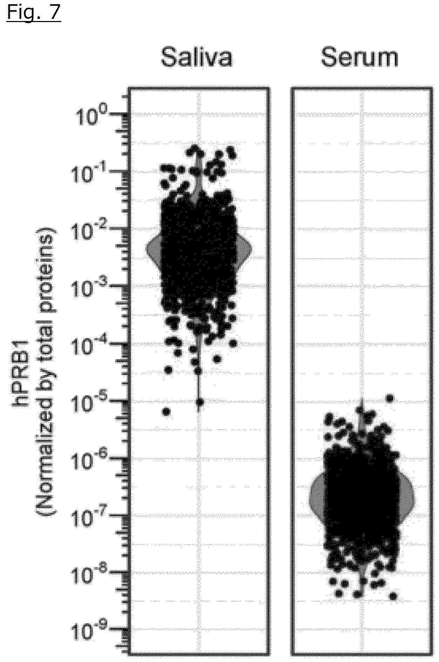

As a result of an analysis, it was found that the hPRB1 constitutes about 0.001 to 30% by weight of total salivary protein (left panel of FIG. 7). Since total protein concentration of the human saliva is about 2 mg/mL, a concentration of the hPRB1 in the human saliva is 0.02 to 600 µg/mL. In addition, the hPRB1 was also detected in the serum samples, although it was previously believed that the hPRB1 was not present in the serum. However, a concentration of the hPRB1 in the serum was much lower than that in the saliva. It was found that the hPRB1 constitutes about 0.0000003 to 0.001% by weight of total serum protein (right panel of FIG. 7). Since the total protein concentration of the human serum is about 80 mg/mL, a concentration of the hPRB1 in the human serum is 0.24 to 800 ng/mL.

The hPRB1 in the saliva could also be detected by the Western blotting using the 8A10 mAb as a detection antibody. The hPRB1 in the serum could also be detected by a pull-down assay using the 8A10 mAb.

### 5. Association of PRB1 in saliva and serum with human CKD

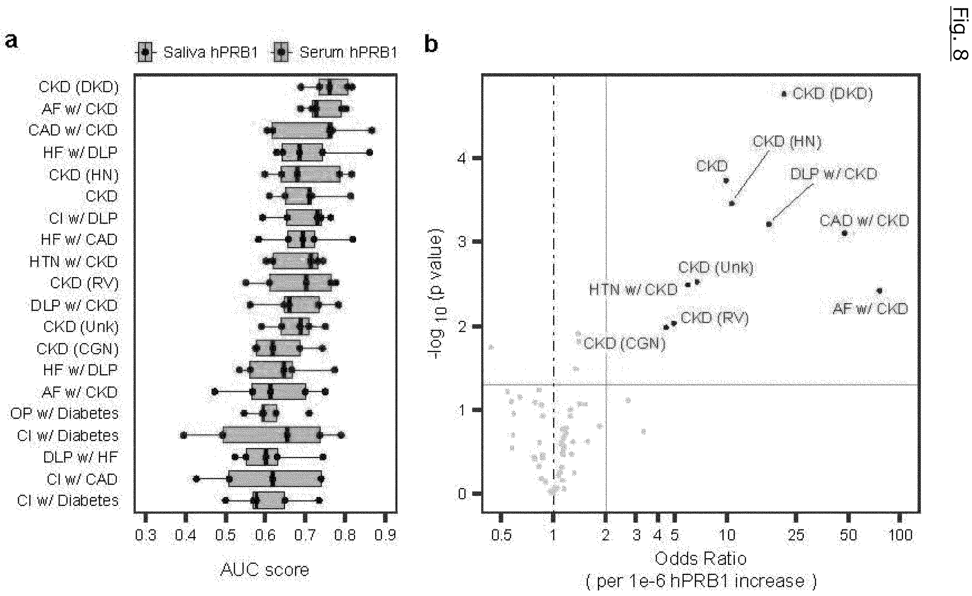

First, the hPRB1 in the saliva and serum were measured in 959 patients with various types of renal disease including chronic kidney disease (CKD), cardiovascular disease, or metabolic disease to assess whether saliva and/or serum hPRB1 levels can correctly distinguish following diseases, including the CKD, using a LightGBM classifier. As a result of an analysis using the LightGBM classifier, it was shown that AUC scores were greater than 0.7 for CKD (diabetic kidney disease: DKD) vs. diseases other than CKD (using the serum HPRB1), atrial fibrillation (AF) with CKD vs. AF without CKD (using the serum hPRB1), coronary artery disease (CAD) with CKD vs. CAD without CKD (using the serum hPRB1), heart failure (HF) with dyslipidemia (DLP) vs. HF without DLP (using the saliva hPRB1), and CKD vs. no CKD (using the serum hPRB1) (FIG. 8a).

Next, a logistic regression model was constructed to calculate odds ratios for the serum hPRB1 levels and each disease type. As a result, odds ratios > 2 and p-values < 0.05 were obtained for the following disease types (FIG. 8b): CKD (DKD) vs. diseases other than CKD, CKD vs. diseases other than CKD, CKD (hypertensive nephrosclerosis: HN) vs. diseases other than CKD, CKD with DLP vs. CKD without DLP, CKD with CAD vs. CKD without CAD, CKD (Unknown: Unk) vs diseases other than CKD, CKD with hypertension (HTN) vs. CKD without HTN, CKD with AF vs. CKD without AF, CKD (renal vasculitis: RV) vs. diseases other than CKD, and CKD(chronic glomerulonephritis: CGN) vs. diseases other than CKD. These results indicate that the serum hPRB1 concentration assumed by the above ELISA system is suitable for assessing CKD-related symptoms.

## Claims

1. An antibody or an antigen-binding fragment thereof having a capability of binding to a human PRB1 protein, wherein
a heavy chain variable region comprises an amino acid sequence that is 95% or more identical to a heavy chain variable region represented by an amino acid sequence from 19th to 132nd positions in SEQ ID NO: 1, and
a light chain variable region comprises an amino acid sequence that is 95% or more identical to a light chain variable region represented by an amino acid sequence from 21st to 132nd positions in SEQ ID NO: 6.

2. The antibody or the antigen-binding fragment thereof according to claim 1, wherein
the heavy chain variable region comprises
a heavy chain CDR1 consisting of an amino acid sequence represented by SEQ ID NO: 3,
a heavy chain CDR2 consisting of an amino acid sequence represented by SEQ ID NO: 4, and
a heavy chain CDR3 consisting of an amino acid sequence represented by SEQ ID NO: 5, and
the light chain variable region comprises
a light chain CDR1 consisting of an amino acid sequence represented by SEQ ID NO: 8,
a light chain CDR2 consisting of an amino acid sequence represented by SEQ ID NO: 9, and
a light chain CDR3 consisting of an amino acid sequence represented by SEQ ID NO: 10.

3. A polynucleotide encoding a polypeptide comprising a heavy chain variable region comprising an amino acid sequence that is 95% or more identical to a heavy chain variable region represented by an amino acid sequence from 19th to 132nd positions in SEQ ID NO: 1.

4. The polynucleotide according to claim 3, wherein
the heavy chain variable region comprises
a heavy chain CDR1 consisting of an amino acid sequence represented by SEQ ID NO: 3,
a heavy chain CDR2 consisting of an amino acid sequence represented by SEQ ID NO: 4, and
a heavy chain CDR3 consisting of an amino acid sequence represented by SEQ ID NO: 5.

5. A vector comprising the polynucleotide according to claim 3.

6. A vector comprising the polynucleotide according to claim 4.

7. A cell expressing a polypeptide comprising a heavy chain variable region comprising an amino acid sequence that is 95% or more identical to a heavy chain variable region represented by an amino acid sequence from 19th to 132nd positions in SEQ ID NO: 1.

8. The cell according to claim 7, wherein
the heavy chain variable region comprises
a heavy chain CDR1 consisting of an amino acid sequence represented by SEQ ID NO: 3,
a heavy chain CDR2 consisting of an amino acid sequence represented by SEQ ID NO: 4, and
a heavy chain CDR3 consisting of an amino acid sequence represented by SEQ ID NO: 5.

9. A polynucleotide encoding a polypeptide comprising a light chain variable region comprises an amino acid sequence that is 95% or more identical to a light chain variable region represented by an amino acid sequence from 21st to 132nd positions in SEQ ID NO: 6.

10. The polynucleotide according to claim 9, wherein
the light chain variable region comprises
a light chain CDR1 consisting of an amino acid sequence represented by SEQ ID NO: 8,
a light chain CDR2 consisting of an amino acid sequence represented by SEQ ID NO: 9, and
a light chain CDR3 consisting of an amino acid sequence represented by SEQ ID NO: 10.

11. A vector comprising the polynucleotide according to claim 9.

12. A vector comprising the polynucleotide according to claim 10.

13. A cell expressing a polypeptide comprising a light chain variable region comprises an amino acid sequence that is 95% or more identical to a light chain variable region represented by an amino acid sequence from 21st to 132nd positions in SEQ ID NO: 6.

14. The cell according to claim 13, wherein
the light chain variable region comprises
a light chain CDR1 consisting of an amino acid sequence represented by SEQ ID NO: 8,
a light chain CDR2 consisting of an amino acid sequence represented by SEQ ID NO: 9, and
a light chain CDR3 consisting of an amino acid sequence represented by SEQ ID NO: 10.

15. The antibody or the antigen-binding fragment thereof according to claim 1 labeled with a labeling substance.

16. A solid phase with immobilized an antibody or an antigen-binding fragment thereof according to claim 1.
